# EUROPEAN PATENT SPECIFICATION

(11) **EP 3 520 845 B1**
(45) Date of publication and mention of the grant of the patent: **17.04.2024**
(21) Application number: 17856390.4
(22) Date of filing: 28.09.2017
(51) Int. Cl.: A61M 5/32, A61M 5/00, A61M 5/31

(54) **SYRINGE ASSEMBLY, PREFILLED SYRINGE, SEAL CAP FOR BARREL WITH PUNCTURE NEEDLE, AND SYRINGE ASSEMBLY PACKAGE**
SPRITZENANORDNUNG, VORGEFÜLLTE SPRITZE, VERSCHLUSSKAPPE FÜR ZYLINDER MIT PUNKTIONSNADEL UND SPRITZENANORDNUNGSVERPACKUNG
ENSEMBLE SERINGUE, SERINGUE PRÉ-REMPLIE, CAPUCHON D'ÉTANCHÉITÉ POUR CORPS DE SERINGUE AVEC AIGUILLE DE PONCTION, ET EMBALLAGE D'ENSEMBLE SERINGUE

(30) Priority: 28.09.2016 JP 2016190181
(43) Date of publication of application: 07.08.2019
(73) Proprietor: Terumo Kabushiki Kaisha, Tokyo 151-0072 (JP); Shin-Etsu Polymer Co., Ltd., Chiyoda-ku Tokyo 101-0041 (JP)
(72) Inventor: KONDO, Keiko, Ashigarakami-gun Kanagawa 259-0151 (JP); ENOMOTO Kaori, Ashigarakami-gun Kanagawa 259-0151 (JP); ABE Yoshihiko, Ashigarakami-gun Kanagawa 259-0151 (JP); NAKAYAMA Kyoko, Kodama-gun Saitama 367-0241 (JP); SHIBA Toru, Kodama-gun Saitama 367-0241 (JP); YOKOYAMA Yusuke, Kodama-gun Saitama 367-0241 (JP); OKUMA Tadashi, Tokyo 101-0041 (JP)
(74) Representative: TBK
(86) International application number: PCT/JP2017/035351
(87) International publication number: WO 2018/062441

(56) References cited:
- WO-A1-2011/114917
- WO-A1-2014/141471
- WO-A1-2016/039111
- WO-A1-2016/051901
- WO-A1-2016/051989
- US-A- 5 538 793

## Description

### TECHNICAL FIELD

The present invention relates to a seal cap for a piercing needle-attached barrel according to the preamble of claim 1, the features of which are know from document WO 2016/051989 A1.

### BACKGROUND ART

As a syringe such as an insulin syringe for administering a small amount of a medical agent to a patient, a syringe having a piercing needle fixed to the distal end portion of a barrel is used. In constructing a prefilled syringe in which the medical agent is filled in advance by using a syringe of this type, it is necessary to seal the tip of the syringe. Seal caps capable of sealing the tip of the syringe are proposed, as disclosed in document WO 2009/016428 A1, document US 6719732 B2, and document JP 2013-43957 A.

Document WO 2016/051989 A1 discloses a Syringe Assembly, pre-Filled Syringe, Seal Cap for Syringe with Puncture Needle, and Syringe Assembly Package in which a mold release agent (lubricant) is blended in a seal cap material.

Document US 5 538 793 A pertains to Silicone rubber particles coated with silicone resin. It is discussed that silicone fine particles improve internal stress reduction and lubricity by blending with various substrates.

### SUMMARY OF THE INVENTION

### PROBLEMS TO BE SOLVED BY THE INVENTION

The seal cap (shield 10) of document WO 2009/016428 A1 covers the tip of the syringe (a part of the injection device is shown in Fig. 2 of patent document 1). The tip of the syringe 3 has the hub 2 to which the needle 6 is fixed. The shield 10 has the open proximal end 11, the closed tip 12, and the wall 13 extended from the proximal end 11 to the closed tip 12. The inner surface 14 of the wall 13 defines the cavity 15 accommodating a part of the tip of the syringe 3. For example, in a case where the shield 10 is fixed to the tip of the syringe to protect the tip while an injection device is being transported before use, the portion 14a of the inner surface 14 contacts the hub 2 disposed at the tip of the syringe 3.

As shown in Figs. 1 through 5 of document US 6719732 D2, the seal cap (device for protecting the syringe needle) of the patent document 2 extends in a longitudinal direction between an open proximal end 22 and a closed distal end 24. The elastic needle cap 20 defines an inner housing 26 delimited by a lateral wall 28 and by an end wall 30. Between the first and second portions 40 and 42 of the housing 26, there is provided an annular bead (rib) 70 forming an inner swell of matter at the level of the end of the second portion 42 facing the proximal end 22. In order to facilitate the passage of the water vapor under pressure and to improve the deformability of this annular bead 70, the bead 70 is provided with four slots 72 extending in longitudinal direction.

As is often the case, the needle-attached prefillable syringe of this type is provided as the prefilled syringe in which a liquid medicine is filled. To seal the needle tip of the barrel, the above-described seal cap is mounted on the barrel. The seal cap is formed by molding an elastic rubber material into which the needle tip can be pierced. To maintain the sealing performance of the prefilled syringe, the needle tip of the prefilled syringe is embedded in the rubber material. Thereby the distal end opening of the needle tip is sealed. In the production process of the prefillable syringe, a sterilizing step is performed. It is preferable to adopt a high-pressure steam sterilization method in the sterilization step. In performing the high-pressure steam sterilization method, to securely sterilize the needle (particularly distal end portion of needle), it is desirable that the seal cap has a high water vapor permeability.

The needle cap disclosed in document JP 2013 - 43957 A is formed of the rubber composition, for protecting the needle mounted on the distal end of the syringe barrel, which contains 70 to 100% by mass of the ethylene-propylene-diene rubber in the base polymer thereof. The seal cap disclosed in the patent document 3 is excellent in that the needle cap has a high water vapor permeability. But it may occur that the material forming the seal cap sticks to the outer surface of the needle tip of the piercing needle, as described later.

The assembly for the syringe of the above-described type is provided in a state where the seal cap has been mounted on the barrel with the piercing needle being pierced into the seal cap. Therefore, the state in which the piercing needle has been pierced into the seal cap, in other words, the state in which the outer surface of the distal end portion of the piercing needle is in close contact with the material forming the seal cap continues for a certain period of time from the time of the production of the assembly for syringe including sterilization process, transport, and storage till the use thereof. To decrease the piercing resistance at a piercing time, normally, silicone is applied to the piercing needle. Nevertheless, in dependence on a storage environment, when the seal cap is removed from the barrel, it may occur that the material forming the seal cap sticks to the outer surface of the piercing needle. The sticking of the material forming the seal cap to the piercing needle may cause an increase of the piercing resistance, which is undesirable.

It is the object of the present invention to provide an assembly for a syringe in which a material forming a seal cap hardly sticks to a piercing needle even after a certain period of time lapses before the assembly for the syringe is used, a prefilled syringe, a seal cap for a piercing needle-attached barrel, and a package for the assembly for the syringe.

The object of the invention is achieved by a seal cap for a piercing needle-attached barrel according to claim 1. Advantageous embodiments are carried out according to the dependent claims.

A seal cap for a piercing needle-attached barrel for achieving the above-described object has the following form.

The seal cap for the piercing needle-attached barrel comprises a piercing needle-attached barrel including a barrel body having a piercing needle mounting portion and a hollow piercing needle which has a piercing needle tip at a distal end thereof and whose proximal end portion is fixed to the piercing needle mounting portion. The seal cap has a pierceable part into which the piercing needle can be pierced. The seal cap is water vapor permeable. At least the pierceable part of the seal cap is formed of an elastic material containing silicone rubber as a main material thereof, silicone particles, and a lubricant.

### BRIEF DESCRIPTION OF THE DRAWINGS

Fig. 1 is a front view of a prefilled syringe of an embodiment of the present invention.
Fig. 2 is a sectional view taken along a line A-A of Fig. 1.
Fig. 3 is an enlarged sectional view of an assembly of the present invention for a syringe for use in the prefilled syringe shown in Figs. 1 and 2.
Fig. 4 is an enlarged front view of a seal cap of the present invention for a piercing needle-attached barrel for use in the prefilled syringe shown in Figs. 1 and 2 and the assembly for the syringe shown in Fig. 3.
Fig. 5 is a bottom view of the seal cap for the piercing needle-attached barrel shown in Fig. 4.
Fig. 6 is an enlarged sectional view taken along a line B-B of Fig. 4.
Fig. 7 is a front view of the barrel for use in the prefilled syringe shown in Figs. 1 and 2 and the assembly for the syringe shown in Fig. 3.
Fig. 8 is an enlarged sectional view taken along a line C-C of Fig. 7.
Fig. 9 is a perspective view of the barrel shown in Fig. 7.
Fig. 10 is an explanatory view for explaining the action of the seal cap of the present invention for the piercing needle-attached barrel.
Fig. 11 is a perspective view of a package of the present invention for accommodating assemblies for syringes.
Fig. 12 is an explanatory view for explaining an inner form of the package for accommodating assemblies for syringes shown in Fig. 11.
Fig. 13 is a front view of the package for assemblies for syringes shown in Fig. 11.
Fig. 14 is a plan view of the package for assemblies for syringes shown in Fig. 13.
Fig. 15 is an enlarged sectional view taken along a line D-D of Fig. 14.

### MODE FOR CARRYING OUT THE INVENTION

By using the embodiments shown in the drawings, description is made below on a seal cap of the present invention for a piercing needle-attached barrel, an assembly, for a syringe, in which the seal cap for the piercing needle-attached barrel has been mounted on the piercing needle-attached barrel, and a prefilled syringe using the assembly, for the syringe, in which the seal cap for the piercing needle-attached barrel has been mounted on the piercing needle-attached barrel.

A prefilled syringe 1 of the present invention is composed of an assembly 10 for a syringe, a gasket 4 which is accommodated inside the assembly 10 for the syringe and liquid-tightly slidable inside the assembly 10 for the syringe, and a medical agent 8 filled inside a space formed of the assembly 10 for the syringe and the gasket 4.

The assembly 10 of the present invention for the syringe includes a piercing needle-attached barrel 2 composed of a barrel body having a piercing needle mounting portion 22 and a hollow piercing needle 6 which has a piercing needle tip 61 at its distal end and whose proximal end portion is fixed to the piercing needle mounting portion 22; and the seal cap 3 for sealing the piercing needle tip 61. The seal cap 3 has a pierceable part 33 into which the piercing needle tip 61 can be pierced. More specifically, the seal cap 3 has a closed distal end part 31, an open proximal end part 32, a hollow part 30 into which at least a distal end portion of the piercing needle 6 can be inserted, and the pierceable part 33 into which the piercing needle tip 61 inserted into the hollow part 30 can be pierced. The seal cap 3 has the closed distal end part 31, the open proximal end part 32, the hollow part 30 accommodating the distal end portion of the piercing needle 6 including at least the piercing needle tip 61 of the piercing needle 6, and the pierceable part 33 into which the piercing needle tip 61 accommodated inside the hollow part 30 can be pierced. The seal cap 3 is water vapor permeable and is placed in a state in which the piercing needle tip 61 has been pierced into the pierceable part 33 of the seal cap. At least the pierceable part 33 of the seal cap 3 is formed of an elastic material containing silicone rubber as its main material, silicone particles, and a lubricant.

The assembly 10 for the syringe (in other words, piercing needle-attached barrel on which cap is mounted) shown in the drawings has the piercing needle-attached barrel 2 composed of the barrel body having the piercing needle mounting portion 22 at the distal end thereof and the piercing needle 6 which has the piercing needle tip 61 at its distal end and whose proximal end portion is fixed to the piercing needle mounting portion 22 and the seal cap 3 mounted on the piercing needle-attached barrel 2.

The seal cap of this embodiment is so constructed as to cover the entire piercing needle. More specifically, the seal cap 3 has the closed distal end part 31, the open proximal end part 32, the cylindrical hollow part 30 extended from the open proximal end part 32 toward the distal end of the seal cap and being capable of accommodating the piercing needle mounting portion 22 therein, and the pierceable part 33 into which the piercing needle tip 61 of the piercing needle 6 inserted into the hollow part 30 can be pierced. The seal cap 3 is water vapor permeable. The seal cap 3 is mounted on the piercing needle-attached barrel 2 with the piercing needle tip 61 of the piercing needle 6 being pierced into the pierceable part 33 of the seal cap 3. In this embodiment, the seal cap 3 is mounted on the piercing needle-attached barrel 2. It is preferable that the seal cap is mounted on the piercing needle-attached barrel in an airtight state. The open proximal end part 32 of the seal cap 3 of this embodiment is mounted on the distal end portion (more specifically, piercing needle mounting portion 22) of the barrel in an airtight state. Although it is preferable that the seal cap has the above-described closed distal end part, the open proximal end part, and the hollow part, the seal cap may have only the pierceable part. As a sealing member of such a type, a block-shaped one is conceivable.

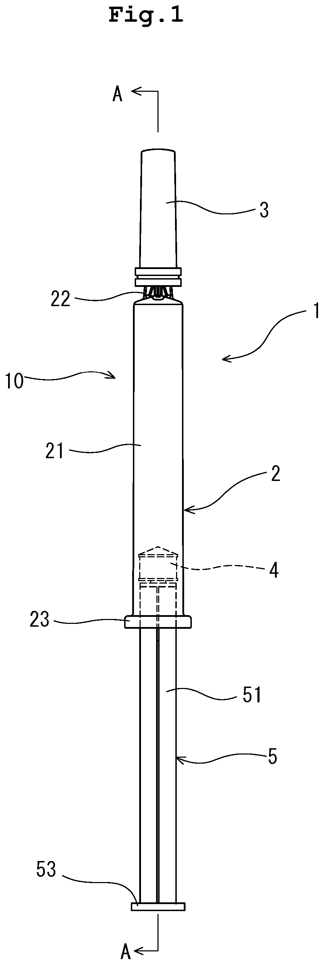

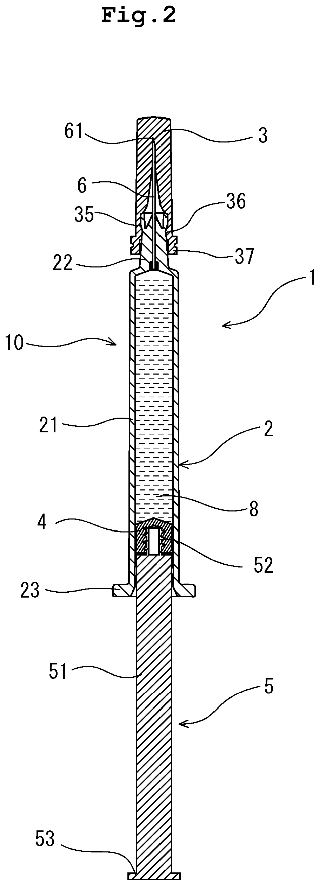

As shown in Figs. 1 and 2, the prefilled syringe 1 is composed of the assembly 10 for the syringe which has the piercing needle-attached barrel 2 and the seal cap 3 mounted on the piercing needle-attached barrel 2 so as to seal the needle tip of the piercing needle, the gasket 4 which is accommodated inside the assembly 10 for the syringe and liquid-tightly slidable inside the assembly 10 for the syringe (inside of the barrel body), the medical agent 8 filled inside the space formed of the assembly 10 for the syringe and of the gasket 4, and a plunger 5 which is mounted on the gasket 4 or to be mounted thereon when the prefilled syringe is used.

The medical agent 8 is filled inside the space formed of the piercing needle-attached barrel 2, the gasket 4, and the inside of the seal cap 3.

As the medical agent 8 to be filled inside the above-described space, it is possible to use any medical agent to be used as injection solutions. Examples of the medical agent include protein pharmaceuticals such as an antibody, peptide pharmaceuticals such as hormone, nucleic acid medicines, cell medicines, blood products, vaccines for protecting infectious diseases, anticancer drugs, anesthetics, jolt, antibiotics, steroid drugs, proteolytic enzyme inhibitor, heparin, sugar injections such as glucose, an electrolyte correction injection solution such as sodium chloride, potassium lactate, and the like, vitamin preparations, fat emulsion, an imaging agent, and a stimulant drug.

The piercing needle-attached barrel 2 has the barrel body composed of a barrel body part 21, the cylindrical (hollow) piercing needle mounting portion 22 provided at the distal end portion of the barrel body part 21, a flange 23 provided at the proximal end portion of the barrel body part 21, and the piercing needle 6 whose proximal end portion is fixed to the piercing needle mounting portion 22.

The piercing needle 6 is a hollow piercing needle penetrating therethrough from its distal end to proximal end. The piercing needle 6 has the piercing needle tip 61 at its distal end. The proximal end portion of the piercing needle 6 is inserted into a hollow portion of the piercing needle mounting portion 22 and fixed thereto with the inside of the piercing needle 6 communicating with an inner space 20 of the barrel body. In other words, the piercing needle 6 is embedded in the piercing needle mounting portion 22 of the barrel body at a portion thereof (proximal end portion of piercing needle not including proximal end thereof) disposed a little distally from the proximal end thereof. Thus, the hollow portion of the piercing needle mounting portion 22 disappears. As the piercing needle 6, a metallic hollow needle having its tip at its distal end is used. In this embodiment, a lubricant is applied to at least the piercing needle tip 61 of the piercing needle 6. Although the kind of the lubricant to be used is not limited to a specific one, silicone oil is used. By using the lubricant, it is possible to expect a decrease in the piercing resistance of the piercing needle tip at a piercing time.

The piercing needle 6 may be inserted into the hollow portion of the piercing needle mounting portion 22 of the barrel body formed by molding a material in advance and fixed to the piercing needle mounting portion 22 by means of an adhesive agent, thermal welding or the like. The piercing needle 6 may be fixed to the barrel body by insert molding. In the case of the insert molding, by forming the barrel body by molding the material, the piercing needle mounting portion 22 has a cylindrical (hollow) configuration into which the piercing needle 6 has been inserted. More specifically, the proximal end portion of the piercing needle 6 is inserted into the hollow portion of the piercing needle mounting portion 22 and fixed thereto.

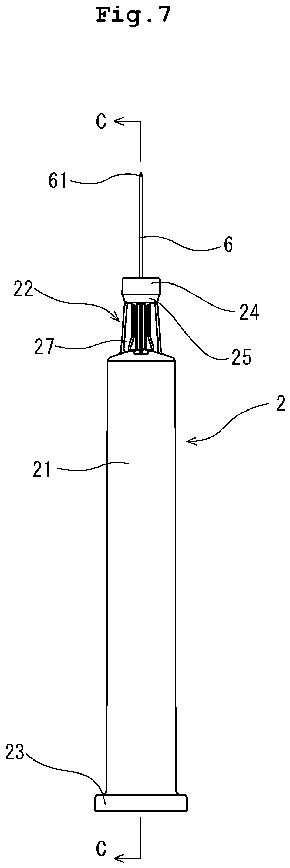

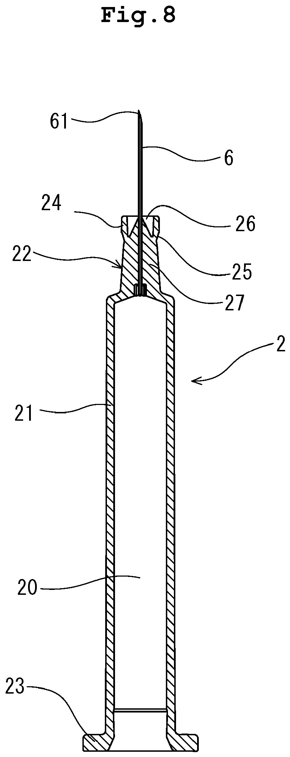

The barrel body of the piercing needle-attached barrel 2 is transparent or semitransparent. The barrel body part 21 of the barrel body is a substantially cylindrical part accommodating the gasket 4 liquid-tightly and slidably. The piercing needle mounting portion 22 is a hollow cylindrical portion projecting forward from the distal end portion (shoulder portion) of the barrel body and having a smaller diameter than the barrel body. As shown in Figs. 7 and 8, the piercing needle mounting portion 22 has a head portion 24 provided at its distal end, a short tapered diameter-decreased portion 25 which is provided at the proximal end of the head portion 24 and becomes shorter toward its proximal end in its diameter, and a connecting portion 27 connecting a proximal end portion of the tapered diameter-decreased portion 25 and the distal end of the barrel body part 21 to each other. A concave portion is formed of the tapered diameter-decreased portion 25. In the head portion 24, there are formed a concavity 26 recessed from a distal end surface of the head portion 24 toward its proximal end and a hollow conical portion which is positioned inside the concavity 26 and has an apex at the distal end side of the head portion 24.

A plurality of grooves extended in the axial direction of the barrel body is formed on an outer surface of the connecting portion 27. It is possible that a concave portion 25 is formed not in a tapered configuration, but has a diameter-decreased configuration so that the concave portion 25 is stepped from the proximal end of the head portion 24. In addition, it is possible to omit the formation of the connecting portion 27 and directly connect the proximal end portion of the concave portion (tapered diameter-decreased portion 25) and the distal end of the barrel body part 21 to each other. It is also possible to omit the formation of the concavity 26 and the conical portion and form the head portion 24 into a hollow and columnar (cylindrical) configuration. It is preferable to form the concave portion 25 annularly as in the case of this embodiment.

As materials for forming the barrel body, various resins including polypropylene, polyethylene, polystyrene, polyamide, polycarbonate, polyvinyl chloride, poly-(4-methylpentene-1), acrylic resin, acrylonitrile-butadiene-styrene copolymer, polyester such as polyethylene terephthalate, cyclic olefin polymer, and cyclic olefin copolymer are exemplified. Of these resins, the polypropylene, the cyclic olefin polymer, and the cyclic olefin copolymer are preferable because these resins are easily moldable and heat-resistant.

As the piercing needle 6, a hollow needle having the piercing needle tip 61 at its distal end is used. As materials for forming the piercing needle 6, metals are normally used. Of metals, stainless steel is preferable.

As shown in Figs. 1 and 2, the gasket 4 has a body part extended in substantially an equal diameter and a plurality of annular ribs (although two annular ribs are formed in this embodiment, not less than two annular ribs may be appropriately formed, provided that the rib satisfies liquid tightness and slidability) formed on the body part. The ribs liquid-tightly contact the inner surface of the barrel body. A distal end surface of the gasket 4 has a configuration corresponding to that of the inner surface of the distal end of the barrel body to prevent the formation of a gap between the distal end surface of the gasket and the inner surface of the distal end of the barrel body as much as possible when both surfaces contact each other.

As materials for forming the gasket 4, it is preferable to use elastic rubber (for example, isoprene rubber, butyl rubber, latex rubber, silicone rubber, and the like); and synthetic resin (for example, styrene elastomer such as SBS elastomer, SEBS elastomer, and the like and olefin elastomer such as ethylene-α-olefin copolymer elastomer).

The gasket 4 has a concave portion extended inward from its proximal end portion. The concave portion of the gasket 4 is female screw-shaped and threadedly engageable with a male screw portion formed on an outer surface of a projected portion 52 formed at a distal end portion of the plunger 5. Threaded engagement between the female and male screw portions prevents the plunger 5 from being removed from the gasket 4. The plunger 5 may be removed from the gasket when the prefilled syringe is not used and mounted thereon when the prefilled syringe is used. The plunger 5 has the projected portion 52 projected forward tubularly from a disk portion disposed at its distal end. The male screw which threadedly engages the concave portion of the gasket 4 is formed on the outer surface of the projected portion. The plunger 5 has a body part 51 cross-shaped in its cross section and axially extended and a pressing disk portion 53 provided at its proximal end portion.

The seal cap 3 of the present invention for the piercing needle-attached barrel is used by mounting the seal cap on the barrel composed of the barrel body part 21, the cylindrical piercing needle mounting portion 22 provided at the distal end portion of the barrel body part 21 and having the head portion 24, and the piercing needle 6 which has the piercing needle tip 61 at its distal end and whose proximal end portion is inserted into the piercing needle mounting portion 22 and fixed thereto.

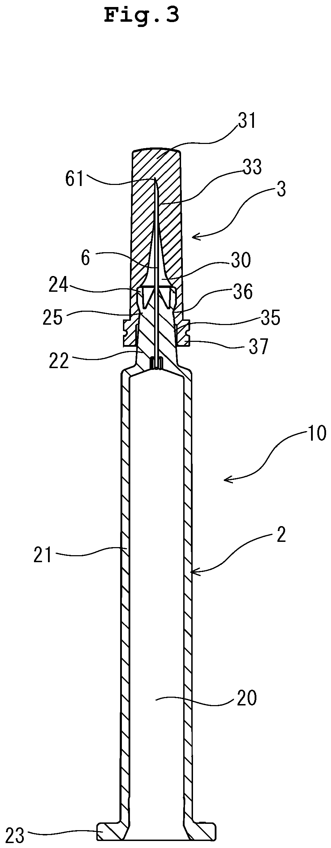

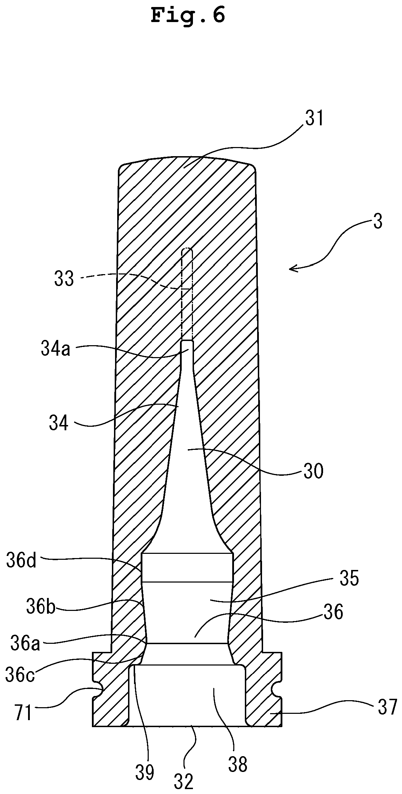

As shown in Fig. 6, the seal cap 3 has the closed distal end part 31, the open proximal end part 32, the hollow part 30, positioned distally from the open proximal end part 32, which has a piercing needle mounting portion accommodating part 35 accommodating the piercing needle mounting portion 22 and a tapered piercing needle accommodating part 34 continuous with the distal end of the piercing needle mounting portion accommodating part 35, the pierceable part 33 into which the piercing needle tip 61 of the piercing needle 6 accommodated inside the piercing needle accommodating part 34 can be pierced, and a projected part 36 formed on the inner surface of the piercing needle mounting portion accommodating part 35. As shown in Fig. 3, in the seal cap 3 of this embodiment mounted on the piercing needle-attached barrel 2, at least a part of the hollow part 30 (more specifically, the piercing needle mounting portion accommodating part 35 which is a part of the hollow part and the vicinity thereof) is expansively spread outward by the piercing needle mounting portion 22 accommodated inside the hollow part 30. The seal cap does not need to have the piercing needle mounting portion accommodating part. In this case, it is preferable that the open end of the open proximal end part of the seal cap contacts (desirably, airtightly contacts) the distal end surface of the piercing needle mounting portion of the barrel.

The seal cap 3 is permeable to water vapor. More specifically, the seal cap is entirely formed of a material permeable to water vapor. At least the pierceable part 33 of the seal cap 3 is formed of an elastic material containing silicone rubber as its main material, silicone particles, and a lubricant. In this embodiment, the seal cap 3 is entirely formed of the elastic material containing the silicone rubber as its main material, the silicone particles, and the lubricant.

It is necessary that the seal cap is entirely formed of the material permeable to water vapor and that the pierceable part 33 thereof is formed of the elastic material containing the silicone rubber as its main material, the silicone particles, and the lubricant. Thus, parts of the seal cap 3 other than the pierceable part 33 may be formed of an elastic material not containing the silicone particles and the lubricant or other materials such as elastomers or rubber compositions permeable to water vapor.

It is preferable that the silicone rubber composing the main material of the seal cap 3 is crosslinked. Crosslinking can be performed by adding a crosslinking agent to a silicone rubber material to crosslink the silicone rubber. A catalyst may be added to the silicone rubber material as necessary.

As a main component of the silicone rubber, organopolysiloxane is used. As the organopolysiloxane, diorganopolysiloxane expressed by a general formula of R¹ₙSiO_{4-n/2} is preferable (in the formula, R¹ denotes an independently unsubstituted or substituted monovalent hydrocarbon group, and n denotes 1.98 to 2.02). Examples of R¹ include an alkyl group such as a methyl group, an ethyl group, a propyl group, and a butyl group; an alkenyl group such as a vinyl group, an allyl group, a propenyl group, a butenyl group, and a hexenyl group; an aryl group such as a phenyl group, a tolyl group, and a xylyl group; an aralkyl group such as a benzyl group and a 2-phenylethyl group; and an identical or different unsubstituted or substituted monovalent hydrocarbon group, having a carbon number of 1 to 10, preferably 1 to 8, which is selected from among a chloromethyl group, a trifluoropropyl group or a cyanoethyl group formed by substituting a part or all of hydrogen atoms bonded with carbon atoms of the above-described groups with halogen atoms, a cyano group or the like. As the substituent group R1 which bonded to the silicon atom, it is possible to use anything of the substituent groups described above basically. As the alkenyl group, the vinyl group is preferable. As the other substituent group, the methyl group or the phenyl group is preferable. In particular, when solvent-resistant are necessary, it is preferable to use a trifluoropropyl group.

It is most favorable that the main chain of the organopolysiloxane consists of linear diorganopolysiloxane. The organopolysiloxane may partly contain a branch structure such as an R¹ₙSiO_{3/2} unit (R¹ is as described above) or a SiO₂ unit. As the main component of the silicone rubber, silicone resin may be used in addition to the organopolysiloxane.

As the lubricant to be added to the elastic material to be used in the present invention, it is possible to use at least one kind selected from among metal soap, higher fatty acid, higher aliphatic alcohol, higher aliphatic amine, and higher aliphatic amide. The content of the lubricant at a lubricant-added portion is preferably 0.3 to 5.0 wt% and especially preferably 0.5 to 3.0 wt%.

As the metal soap, metal salts (metals other than alkali metal) of long-chain fatty acids and those of organic acids are used. Specifically, it is possible for the metal soap to use zinc stearate, calcium stearate, barium stearate, lithium stearate, magnesium stearate, calcium laurate, barium laurate, zinc laurate, calcium ricinoleate, barium ricinoleate, zinc ricinoleate, and zinc octylate.

As the higher fatty acid, it is possible to use saturated or unsaturated higher fatty acid such as lauric acid, myristic acid, palmitic acid, stearic acid, oleic acid, arachidic acid, and behenic acid.

As the higher aliphatic amides, it is possible to use fatty acid amide such as amide stearate, amide oleate and erucic acid amide and alkylene fatty acid amide such as methylene bis-stearic acid amide and ethylene bis-stearic acid amide.

As the higher aliphatic amine, it is possible to use aliphatic primary amines such as lauryl amine, myristyl amine, palmityl amine, stearyl amine, oleyl amine and aliphatic tertiary amines such as long chain alkyl dimethyl tertiary amines having lauryl, myristyl, palmityl, stearyl and the like. As the higher fatty alcohol, it is possible to use stearyl alcohol and the like. As esters of the higher fatty alcohol, it is possible to use esters of stearyl alcohol.

As the silicone particle, it is possible to use spherical silicone particles and amorphous silicone particles. The spherical silicone particles are preferable. As the silicone particle, fine particles whose size (d50) measured by a laser diffraction method at 50% in cumulative frequency is 0.5 to 50µm are preferable. Fine particles whose size is 0.7 to 40µm are especially favorable. The content of the silicone particle at a silicone particle-added portion is preferably 0.5 to 10 wt% and especially preferably 1 to 5 wt%.

As the silicone particle, at least one kind selected from among silicone rubber particles, silicone resin particles, and silicone composite particles is preferable.

As the silicone rubber particles, it is possible to use silicone elastomer powder having a structure composed mainly of the linear crosslinked organopolysiloxane. For example, the silicone elastomer powder is described in Japanese Patent Application Laid-Open Publication No. H03(1991)-93834. As the silicone resin particles, it is possible to use fine powder of polyorganosilsesquioxane. For example, the fine powder of polyorganosilsesquioxane is described in Japanese Patent Application Laid-Open Publication No. H02(1990)-209927. For example, the silicone composite particles consisting of silicone rubber powder whose surface is coated with silicone resin is described in Japanese Patent Application Laid-Open Publication No. H07(1995)-196815 (USP 5538793). The silicone particles may consist of any one of the particles selected from among the above-described particles or mixtures thereof.

As the silicone rubber particles, cured materials of the organopolysiloxane having the above-described linear organopolysiloxane block are exemplified. R¹ in the above-described formula denotes a substituted or unsubstituted monovalent hydrocarbon group having a carbon number of 1 to 30, preferably 1 to 20. Examples of R¹ include an alkyl group such as a methyl group, an ethyl group, a propyl group, a butyl group, a pentyl group, a hexyl group, a heptyl group, an octyl group, a decyl group, a undecyl group, a dodecyl group, a tetradecyl group, a pentadecyl group, a hexadecyl group, a heptadecyl group, an octadecyl group, a nonadecyl group, an icosyl group (=eicosyl group), a henicosyl group (=heneicosyl group), a docosyl group, a tricosyl group, a tetradecyl group, and a triacontyl group; an aryl group such as a phenyl group, a tryl group, and a naphtyl group; an aralkyl group such as a benzyl group, a phenetyl group; an alkenyl group such as a vinyl group and an aryl group; a cycloalkyl group such as a cyclopentyl group, a cyclohexyl group, a cycloheptyl group; and groups formed by substituting a part or all of hydrogen atoms bonded with carbon atoms of the above-described groups with halogen atoms (fluorine atom, chloride atom, bromide atom, and iodine atom) and/or an acryloyloxy group, a methacryloyloxy group, an epoxy group, a glycidoxy group, a carboxyl group or the like. Among these groups, the methyl group is preferable. Reference symbol n denotes integers of 5 to 5,000, preferably integers of 10 to 3,000.

More specifically, examples of the silicone rubber particles include KMP-597 produced by Shin-Etsu Chemical Co., Ltd. (having a structure obtained by crosslinking linear organopolysiloxane, spherical, average particle size: 5µm, particle size distribution: 1 to 10µm, true specific gravity: 0.97), KMP-598 produced by Shin-Etsu Chemical Co., Ltd. (having a structure obtained by crosslinking linear organopolysiloxane, spherical, average particle size: 13µm, particle size distribution: 2 to 30µm, true specific gravity: 0.97), and KMP-594 produced by Shin-Etsu Chemical Co., Ltd. (having a structure obtained by crosslinking linear organopolysiloxane, spherical, average particle size: 5µm, particle size distribution: 1 to 10µm, true specific gravity: 0.97).

In a case where the silicone rubber particles are used, it is preferable that the silicone rubber forming the silicone rubber particle is different from the silicone rubber to be used as the main material.

As an example of the silicone resin particles, it is possible to use polyorganosilsesquioxane having a crosslinked three-dimensional network structure represented by (RSiO_{3/2})ₙ. Examples of the silicone resin particles include KMP-590 produced by Shin-Etsu Chemical Co., Ltd. (spherical, average particle size: 2µm, particle size distribution: 1 to 4um, true specific gravity: 1.3), KMP-701 produced by Shin-Etsu Chemical Co., Ltd. (spherical, average particle size: 3.5um, particle size distribution: 1 to 6µm, true specific gravity: 1.3), X-52-854 produced by Shin-Etsu Chemical Co., Ltd. (spherical, average particle size: 0.7µm, particle size distribution: 0.2 to 5um, true specific gravity: 1.3), and X-52-1621 produced by Shin-Etsu Chemical Co., Ltd. (spherical, average particle size: 5µm, particle size distribution: 1 to 8um, true specific gravity: 1.3).

The silicone composite particles consisting of silicone rubber powder whose surfaces are coated with the silicone resin are preferable. Examples of the silicone composite particles having such a structure include KMP-600 produced by Shin-Etsu Chemical Co., Ltd. [spherical, average particle size: 5µm, particle size distribution: 1 to 15µm, true specific gravity: 0.99, and rubber hardness (durometer A)30], KMP-601 produced by Shin-Etsu Chemical Co., Ltd. [spherical, average particle size: 12µm, particle size distribution: 2 to 25µm, true specific gravity: 0.98, and rubber hardness (durometer A)30], KMP-602 produced by Shin-Etsu Chemical Co., Ltd. [spherical, average particle size: 30µm, particle size distribution: 4 to 60µm, true specific gravity: 0.98, and rubber hardness (durometer A)30], KMP-605 produced by Shin-Etsu Chemical Co., Ltd. [spherical, average particle size: 2µm, particle size distribution: 0.7 to 5µm, true specific gravity: 0.99, and rubber hardness (durometer A)75], and X-52-7030 produced by Shin-Etsu Chemical Co., Ltd. [spherical, average particle size: 0.8µm, particle size distribution: 0.2 to 2µm, true specific gravity: 1.01, and rubber hardness (durometer A)75].

As the silicone particles, in addition to the above-described examples, it is possible to use Trefil E-5500 (trade name) produced by Dow Corning Toray Co., Ltd. (silicone rubber powder, spherical, average particle size: 3µm, true specific gravity: 0.97), Trefil E-606 (trade name) produced by Dow Corning Toray Co., Ltd. (silicone rubber powder, spherical, average particle size: 2um, true specific gravity: 0.98), Tospearl 120 (trade name) produced by Momentive Performance Materials Japan LLC (silicone resin fine particles, spherical, average particle size: 2µm, true specific gravity: 1.32), Tospearl 130 (trade name) produced by Momentive Performance Materials Japan LLC (silicone resin fine particles, spherical, average particle size: 3µm, true specific gravity: 1.32), and Tospearl 145 (trade name) produced by Momentive Performance Materials Japan LLC (silicone resin fine particles, spherical, average particle size: 4.5um, true specific gravity: 1.32).

The open proximal end part 32 of the seal cap 3 of this embodiment is mounted on the distal end portion of the barrel 2. The seal cap 3 has the piercing needle mounting portion accommodating part 35, located distally with respect to the open proximal end part 32, which accommodates the distal end portion of the piercing needle mounting portion 22 of the barrel body. The inner surface of the piercing needle mounting portion accommodating part 35 of the seal cap 3 and the outer surface of the piercing needle mounting portion 22 of the barrel body are kept in close contact with each other.

The form of the inner surface of the seal cap 3 of this embodiment is described below.

The seal cap 3 has the projected part 36 formed on the inner surface thereof located distally with respect to the open proximal end part 32 at a predetermined length. The projected part 36 has an apex 36a projected to a highest extent and an inclined portion (tapered portion) 36b which is extended from the apex 36a toward the distal end of the seal cap and becomes gradually lower in its projection height toward the distal end of the seal cap. In this embodiment, the projected part 36 is formed as an annular projected part. The inclined portion 36b is formed as a tapered portion in such a way that the inner diameter of the piercing needle mounting portion accommodating part 35 decreases gradually toward the distal end of the seal cap.

The inner diameter of the piercing needle mounting portion accommodating part 35 at the apex 36a is set a little smaller than the outer diameter of the head portion 24 of the piercing needle mounting portion 22 of the barrel body. Thereby when the seal cap 3 is mounted on the piercing needle mounting portion 22 of the piercing needle-attached barrel 2, the piercing needle mounting portion accommodating part 35 which forms a part of the hollow part and the vicinity thereof are expansively spread outward by the piercing needle mounting portion 22 accommodated inside the hollow part 30, as shown in Fig. 3.

Thereby the inner surface of the piercing needle mounting portion accommodating part 35 of the seal cap 3 is pressed against the outer surface of the head portion 24 with both surfaces kept in close contact with each other. Further, in this embodiment, the projected part 36 of the seal cap 3 and the concave portion 25 are kept in engagement with each other. In a state where the seal cap 3 is mounted on the piercing needle mounting portion 22 of the piercing needle-attached barrel 2, the distal end side inclined portion 36b is extended distally with respect to the concave portion 25. The inner diameter of the piercing needle mounting portion accommodating part 35 at least at the distal end portion of the distal end side inclined portion 36b is set a little smaller than the outer diameter of the head portion 24 of the piercing needle mounting portion 22 of the barrel body.

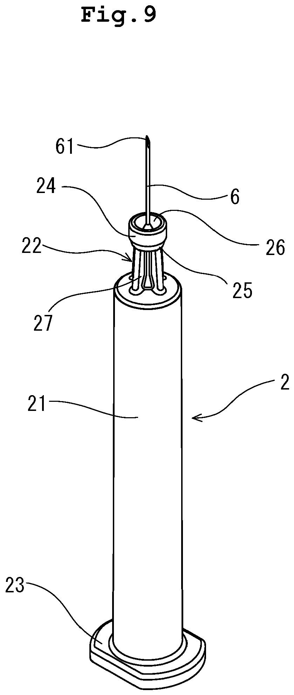

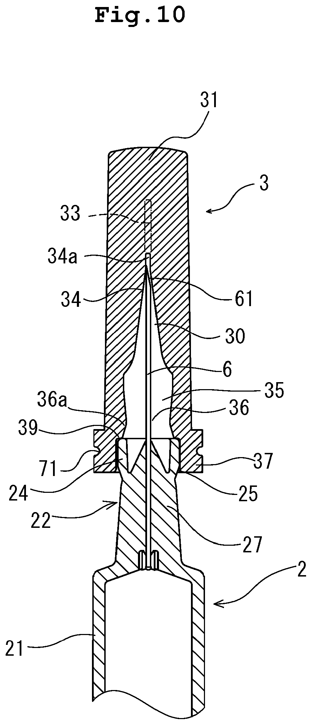

Thereby as shown in Figs. 9 and 10, when the seal cap 3 is mounted on the piercing needle mounting portion 22 of the piercing needle-attached barrel 2, the inner surface of the distal end side inclined portion 36b is entirely pressed against the outer surface of the head portion 24 with both surfaces kept in close contact with each other. Thereby it is possible to reduce undesired removal of the seal cap 3 from the piercing needle-attached barrel 2 to a higher extent. Further, because the distal end side inclined portion 36b is pressed against the outer surface of the head portion 24, at least the piercing needle mounting portion accommodating part 35 is placed in a state in which it is expansively spread outward by the heat portion 24.

In the seal cap 3 of this embodiment, as shown in Fig. 6, the projected part 36 further includes a proximal end side inclined portion 36c which extends from the apex 36a toward the open end (proximal end) of the seal cap and gradually decreases in its projection height toward the open end (proximal end) of the seal cap. Thereby in mounting the seal cap 3 on the piercing needle mounting portion 22 of the piercing needle-attached barrel 2, the apex 36a of the projected part 36 is capable of easily climbing over the head portion 24 of the piercing needle mounting portion 22 from the distal end side of the head portion 24.

In the seal cap 3 of this embodiment, the piercing needle mounting portion accommodating part 35 has a linear portion 36d extended at a predetermined length (more specifically, extended to the proximal end portion of the piercing needle accommodating part 34) from a distal end portion of the distal end side inclined portion 36b of the projected part 36 toward the distal end of the seal cap. The inner diameter of the piercing needle mounting portion accommodating part 35 at the linear portion 36d thereof is set constant and a little smaller than the outer diameter of the head portion 24 of the piercing needle mounting portion 22 of the barrel body. Thereby when the seal cap 3 is mounted on the piercing needle mounting portion 22 of the piercing needle-attached barrel 2, the linear portion 36d is pressed against the outer surface of the head portion 24 with the linear portion 36d kept in close contact with the outer surface of the head portion 24. Because the linear portion 36d is pressed against the outer surface of the head portion 24, at least the piercing needle mounting portion accommodating part 35 is placed in the state in which it is expansively spread outward by the heat portion 24.

In this embodiment, the proximal end portion of the distal end side inclined portion 36b of the projected part 36 of the seal cap 3 is positioned on the periphery of the concave portion 25 of the piercing needle mounting portion 22 of the piercing needle-attached barrel 2. The inner diameter of the piercing needle mounting portion accommodating part 35 at least in the vicinity of the proximal end portion of the distal end side inclined portion 36b is a little smaller than the outer diameter of the annular concave portion 25. Thereby, the distal end side inclined portion 36b of the projected part 36 of the seal cap 3 is pressed against the outer surface of the concave portion 25 with the distal end side inclined portion 36b kept in close contact with the outer surface of the concave portion 25. Therefore, it is possible to reduce undesired removal of the seal cap 3 from the piercing needle-attached barrel 2 to a higher extent.

In this embodiment, the concave portion 25 consists of the tapered diameter-decreased portion which is provided at the proximal end side of the head portion 24 and decreases toward the proximal end thereof in its diameter. Thereby in removing the seal cap 3 from the piercing needle-attached barrel 2, the projected part 36 of the seal cap 3 is expansively spread outward along the concave portion 25 and is thus capable of easily climbing over the head portion 24.

In the seal cap 3 of this embodiment, the hollow part 30 has a piercing needle mounting portion-introducing portion 38 which is formed in a range from the open proximal end part 32 of the seal cap 3 to the proximal end of the piercing needle mounting portion accommodating part 35 (projected part 36) and is extended in a substantially equal inner diameter. The piercing needle mounting portion-introducing portion 38 has an inner diameter a little larger than a maximum inner diameter of the piercing needle mounting portion accommodating part 35 and a little larger than the outer diameter of the head portion 24 of the piercing needle mounting portion 22 of the piercing needle-attached barrel 2. Therefore, in mounting the seal cap 3 on the piercing needle mounting portion 22 of the piercing needle-attached barrel 2, the piercing needle mounting portion-introducing portion 38 functions as a portion for introducing the piercing needle mounting portion 22 into the seal cap.

The piercing needle mounting portion-introducing portion 38 has an annular erect surface 39 erect toward the open proximal end part 32 at a boundary between the piercing needle mounting portion-introducing portion 38 and the proximal end of the piercing needle mounting portion accommodating part 35 (projected part 36). Thus, when the distal end portion of the piercing needle-attached barrel 2 is inserted into the piercing needle mounting portion-introducing portion 38 of the seal cap 3, the piercing needle mounting portion 22 of the piercing needle-attached barrel 2 enters the piercing needle mounting portion-introducing portion 38. Thereafter as shown in Fig. 10, an annular distal end surface of the head portion 24 of the piercing needle mounting portion 22 contacts the annular erect surface 39. In this state, the piercing needle 6 becomes substantially parallel with the central axis of the seal cap 3 and enters a small-diameter distal end portion 34a of the piercing needle accommodating part 34.

A gripping flange 37 is formed at the proximal end portion of the seal cap 3 by projecting the flange 37 outward and annularly. An annular concave portion 71 is formed on the flange 37. The distal end position of the flange 37 is located distally with respect to the annular erect surface 39 of the hollow part 30 and in the vicinity of the apex 36a of the projected part 36 (in the case of the flange shown in Fig. 6, the distal end position of the flange is located a little nearer to the open proximal end part 32 than the apex 36a).

The method for producing the seal cap of the present invention is described below.

The method for producing the seal cap of the present invention is to produce the seal cap which is mounted on the needle-attached barrel composed of the barrel body having the piercing needle mounting portion at its distal end and the piercing needle which has the piercing needle tip at its distal end and whose proximal end portion is inserted into the piercing needle mounting portion and fixed thereto.

The method for producing the seal cap of the present invention is to prepare the seal cap which has the cylindrical hollow part accommodating the piercing needle mounting portion of the piercing needle-attached barrel therein and the pierceable part into which the piercing needle tip of the piercing needle inserted into the hollow part can be pierced. The seal cap is formed of the elastic material containing the silicone rubber as its main material.

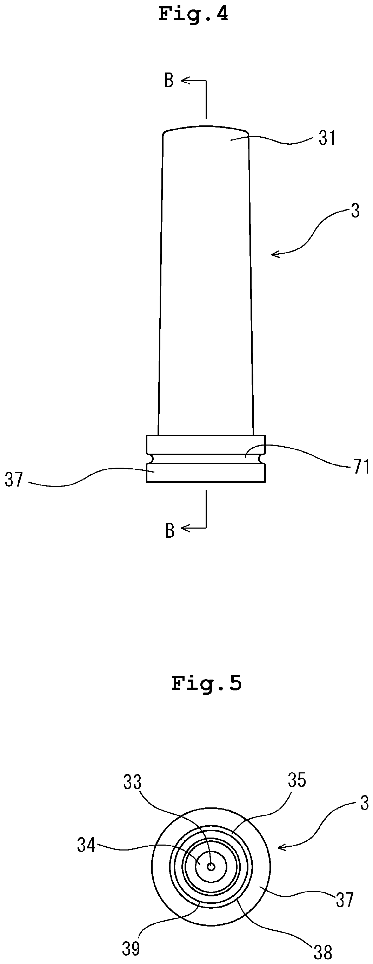

The process of preparing the seal cap is to prepare a seal cap body which has the cylindrical hollow part accommodating the piercing needle mounting portion of the piercing needle-attached barrel therein and the pierceable part into which the piercing needle tip of the piercing needle inserted into the hollow part can be pierced. The seal cap body has the form as shown in Fig. 4 through 6.

The seal cap body to be prepared is produced by using the above-described elastic material as the material for the seal cap.

The method for sterilizing the assembly of the present invention for the syringe is described below.

The method for sterilizing the assembly of the present invention for the syringe is to sterilize the assembly for the syringe including the barrel composed of the barrel body part, the piercing needle mounting portion provided at the distal end portion of the barrel body part, and the piercing needle which has the piercing needle tip at its distal end and whose proximal end portion is fixed to the piercing needle mounting portion, and the seal cap mounted on the barrel.

In a factory for producing a medical container such as a barrel for a prefilled syringe and the like, it is normal that after the medical container is accommodated inside a predetermined accommodating container, the accommodating container accommodating the medical container is put in a sterilization bag and sealed. Thereafter the sterilizing bag is sterilized. By putting the sterilized bag inside an outer packaging container and sealing the outer packaging container, the production of a medical package accommodating the sterilized medical container is completed. The medical package is packed inside a box and shipped. Normally, high-pressure steam sterilization is performed at a temperature not less than 120 degrees C. Therefore, normally, the accommodating container is composed of a material water vapor permeable and bacteria impermeable.

### EXAMPLES

As materials for the seal cap, silicone crude rubber (trade name: "KE-941-U" produced by Shin-Etsu Chemical Co., Ltd.), various kinds of lubricants, and various kinds of silicone particles were prepared. The following lubricants and the silicone particles were prepared.

Lubricant A: zinc stearate
Lubricant B: calcium stearate
Lubricant C: lauric acid amide (produced by Nihon Kasei Co., Ltd., trade name: Diamid Y)
Lubricant D: aliphatic amine (produced by NOF CORPORATION, trade name: Nissan Amine)
Silicone particle A: silicone rubber particle (produced by Shin-Etsu Chemical Co., Ltd., KMP-597)
Silicone particle B: silicone rubber particle (produced by Shin-Etsu Chemical Co., Ltd., KMP-598)
Silicone particle C: silicone resin particle (produced by Shin-Etsu Chemical Co., Ltd., KMP-590)
Silicone particle D: silicone resin particle (produced by Shin-Etsu Chemical Co., Ltd., KMP-701)
Silicone particle E: silicone composite particle (produced by Shin-Etsu Chemical Co., Ltd., KMP-600) obtained by coating the surface of silicone rubber powder with silicone resin
Silicone particle F: silicone composite particle (produced by Shin-Etsu Chemical Co., Ltd., X-52-854) obtained by coating the surface of silicone rubber powder with silicone resin
Silicone particle G: silicone composite particle (produced by Shin-Etsu Chemical Co., Ltd., KMP-602) obtained by coating the surface of silicone rubber powder with silicone resin
Silicone particle H: silicone composite particle (produced by Shin-Etsu Chemical Co., Ltd., X-52-7030) obtained by coating the surface of silicone rubber powder with silicone resin

The lubricants and the silicone particles were added to the silicone crude rubber in accordance with the mixing ratios shown in table 1. After a crosslinking agent and a catalyst were further added to the silicone crude oil, the materials were kneaded by using a closed type kneading machine to obtain unvulcanized rubber compounds. Each of the unvulcanized rubber compounds was pressurized and heated to crosslink it by using a predetermined molding die. In this manner, seal caps having a configuration as shown in Fig. 2 were produced.

**Table 1**

| | Lubricant | | Silicone particle | |
|---|---|---|---|---|
| | Kind | wt% | Kind | wt% |
| Example 1 | A | 0.5 | A | 1 |
| Example 2 | A | 3.0 | A | 5 |
| Example 3 | A | 0.3 | A | 7 |
| Example 4 | B | 0.5 | A | 1 |
| Example 5 | C | 0.5 | A | 1 |
| Example 6 | D | 0.5 | A | 1 |
| Example 7 | A | 0.5 | B | 1 |
| Example 8 | A | 0.5 | C | 1 |
| Example 9 | A | 0.5 | D | 1 |
| Example 10 | A | 0.5 | E | 1 |
| Example 11 | A | 0.5 | F | 1 |
| Example 12 | A | 0.5 | G | 1 |
| Example 13 | A | 0.5 | H | 1 |
| Comparison example 1 | - | 0 | A | 3 |
| Comparison example 2 | A | 1 | - | 0 |

After the above-described lubricant, silicone particles, crosslinking agent, and catalyst shown in table 2 were added to isoprene rubber as materials of seal caps, the materials were kneaded by using the closed type kneading machine to obtain unvulcanized rubber compounds. Each of the unvulcanized rubber compounds was pressurized and heated to crosslink it by using the predetermined molding die. In this manner, seal caps having the configuration as shown in Fig. 2 were produced.

**Table 2**

| | Lubricant | | Silicone particle | |
|---|---|---|---|---|
| | Kind | wt% | Kind | wt% |
| Comparison example 3 | A | 3.0 | A | 30 |

After the above-described silicone particles, crosslinking agent and catalyst shown in table 3 were added to butyl rubber as materials of seal caps, the materials were kneaded by using the closed type kneading machine to obtain unvulcanized rubber compounds. Each of the unvulcanized rubber compounds was pressurized and heated to crosslink it by using the predetermined molding die. In this manner, seal caps having the configuration as shown in Fig. 2 were produced.

**Table 3**

| | Lubricant | | Silicone particle | |
|---|---|---|---|---|
| | Kind | wt% | Kind | wt% |
| Comparison example 4 | - | 0 | A | 30 |

By using a piercing needle made of 29-gauge stainless steel and a cyclic olefin polymer (COP) which is a cyclic olefin homopolymer as resin for forming barrels, needle-attached barrels which have the form as shown in Figs. 7 through 9 and allows the above-described seal caps to be mounted thereon were produced by insert molding. After applying a lubricant (silicone oil) to needle tips of the piercing needles, the seal caps of the examples 1 through 13 and the comparison examples 1 through 4 were mounted on the needle-attached barrels respectively. In this way, syringe assemblies in each of which the needle tip of the piercing needle was pierced into the seal cap were produced. 150 assemblies for syringe of each of the examples and the comparison examples were produced by subjecting them to high-pressure steam sterilization.

### Experiment 1: Sticking Property Checking Test

A sticking property checking test was conducted by using 100 assemblies for syringe of each of the examples 1 through 13 and the comparison examples 1 through 4 produced as described above and sterilized. In the test, the seal cap was removed from each assembly for syringe to check whether the material forming the seal cap stuck to the vicinity of needle tip of the piercing needle by using an optical microscope. Table 4 shows the results. The mark of "o" in the sticking property column of table 4 shows that the material forming the seal cap did not stick to the needle tip (needle shielding rubber piece whose area was larger than 0.03mm² stuck to none of the needle tips). The mark of "×" shows that the material forming the seal cap stuck to the needle tip (needle shielding rubber piece whose area was larger than 0.03mm² stuck to one or more needle tips). It could be confirmed that in the assemblies for syringe using the seal caps of the examples, the material forming the seal caps did not stick to the piercing needle even after they were sterilized.

### Experiment 2: Measurement of Water Vapor Permeability

The unvulcanized rubber compound of each of the examples 1 through 13 and the comparison examples 1 through 4 was vulcanized and molded into a sheet to measure the water vapor permeability of each sheet. To measure the water vapor permeability, a water vapor test was conducted at 125 degrees C in accordance with "Method of determining water vapor permeability of plastic film and sheet" described in JIS K7129 (revised on 2008/03/20). The test results were as shown in table 4. The mark of "o"of table 4 indicates that water vapor permeability coefficients of the sheets are not less than 0.4. The mark of "×" of table 4 in the column indicates that water vapor permeability coefficients of the sheets are less than 0.4. It could be confirmed that the materials forming the seal caps of the examples had satisfactory water vapor permeability.

### Experiment 3: Sterilizing Performance Test

By using the seal cap and needle-attached barrel of each of the examples 1 through 13 and the comparison examples 1 through 4 produced as described above and sterilized, BI bacteria (2.7 × 10⁶CFU/ml) were filled inside each seal cap. Thereafter 20 assemblies for syringe mounted on the barrels were produced. The seal caps each subjected to fungus liquid treatment was placed in a state in which the fungus liquid was applied entirely to the inner surface of the seal cap by dripping the fungus liquid into the seal cap and thereafter drying the fungus liquid. Thereafter the seal cap having the fungus liquid applied entirely to the inner surface thereof was mounted on the needle-attached barrel. The piercing needle tip of the needle (piercing needle) of the barrel was pierced into the pierceable part of the cap mounted on the needle-attached barrel. At this time, the open proximal end part of the seal cap was mounted on the distal end portion of the barrel in an airtight state. When the cap was mounted on the barrel, the needle tip contacted the inner surface of the cap. After the needle tip contacted the pierceable part, the needle tip is pierced thereinto. Thus, it is supposed that bacteria stuck to the needle tip. After the seal cap was mounted on the barrel, each assembly for syringe was subjected to high-pressure steam sterilization treatment (123 degrees C, 80 minutes). After the assembly for syringe was sterilized, the seal cap was aseptically removed from the barrel, and the seal cap and the needle-attached barrel were put in a centrifuge tube containing a culture medium. Bacteria were cultured for seven days to check the growth thereof. The results were as shown in table 4. The mark of "o" in the sterility column of table 4 indicates that the BI bacteria could be sterilized (growth of bacteria did not occur), whereas the mark of "×" indicates that bacteria could not be sterilized (growth of bacteria occurred). It could be confirmed that the seal caps of the examples were steric by high pressure steam. The mark of "o" indicates that both the seal cap and the needle-attached barrel (including piercing needle tip) could be sterilized (bacteria were killed).

### Experiment 4: Removal Strength Test of Seal Cap

By using the seal cap and needle-attached barrel of each of 20 assemblies for syringe f each of the examples 1 through 13 and the comparison examples 1 through 4 produced as described above and sterilized, after silicone was applied to the needle tip of the piercing needle, seal caps were mounted on the barrels respectively at a predetermined position thereof. The internal pressure inside the seal cap rises when the seal cap is mounted on the barrel. Thus, a gap was formed between the barrel and the seal cap by deforming a portion of the seal cap in the vicinity of the open portion thereof so as to release the internal pressure. After the seal cap was mounted on the barrel, the seal cap and the barrel were subjected to high-pressure steam treatment (123 degrees C, 80 minutes). After a hook was put on the distal end of the seal cap, the flange of the barrel was fixed to conduct a tensile test by using an autograph (load cell MAX100N). In this manner, the removal strength of the seal cap was measured. The test results were as shown in table 4. It is favorable that the seal cap has a low removal strength. The mark of "o" in the removal strength column of table 4 indicates that the average value of the removal strengths of 20 seal caps was not more than 2N, whereas the mark of "×" indicates that the average value of the removal strengths thereof was not less than 2N. It could be confirmed that the seal caps of the examples had a low removal strength and thus, had an excellent removability.

### Experiment 5: Piercing Resistance Test

The piercing resistance of each of 20 assemblies for syringe of each of the examples 1 through 13 and the comparison examples 1 through 4 produced in the above-described manner and sterilized was measured. After the seal cap was removed from the barrel, the value of the piercing resistance to the silicone rubber (t=0.5mm) was measured by using an autograph. The results were as shown in table 4. It is favorable that the piercing resistance value is low. The mark of "o" in the piercing resistance column of table 4 indicates that the average of the piercing resistance values of the 20 piercing needles was not more than 0.2N, whereas the mark of "×" indicates that the average of the piercing resistance values thereof was not less than 0.2N. It could be confirmed that the needle tips pierced into the seal caps of the examples respectively had a sufficiently high piercing performance.

**Table 4**

| | Sticking property test | Water vapor permeability | Sterilizing performance | Removability | Piercing resistance |
|---|---|---|---|---|---|
| Example 1 | ○ | ○ | ○ | ○ | ○ |
| Example 2 | ○ | ○ | ○ | ○ | ○ |
| Example 3 | ○ | ○ | ○ | ○ | ○ |
| Example 4 | ○ | ○ | ○ | ○ | ○ |
| Example 5 | ○ | ○ | ○ | ○ | ○ |
| Example 6 | ○ | ○ | ○ | ○ | ○ |
| Example 7 | ○ | ○ | ○ | ○ | ○ |
| Example 8 | ○ | ○ | ○ | ○ | ○ |
| Example 9 | ○ | ○ | ○ | ○ | ○ |
| Example 10 | ○ | ○ | ○ | ○ | ○ |
| Example 11 | ○ | ○ | ○ | ○ | ○ |
| Example 12 | ○ | ○ | ○ | ○ | ○ |
| Example 13 | ○ | ○ | ○ | ○ | ○ |
| Comparison example 1 | × | ○ | ○ | ○ | ○ |
| Comparison example 2 | × | ○ | ○ | ○ | ○ |
| Comparison example 3 | × | ○ | ○ | ○ | ○ |
| Comparison example 4 | ○ | × | × | ○ | ○ |

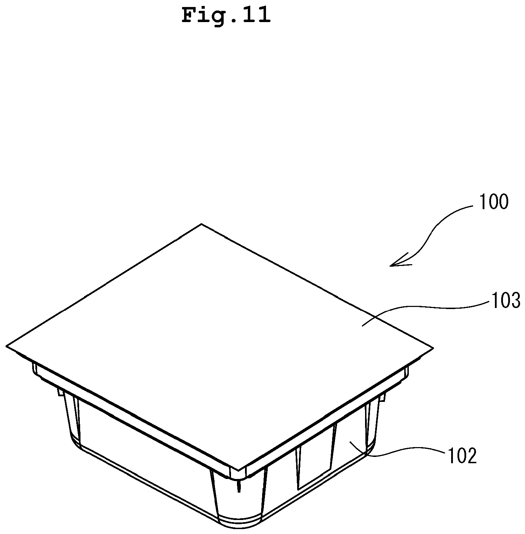

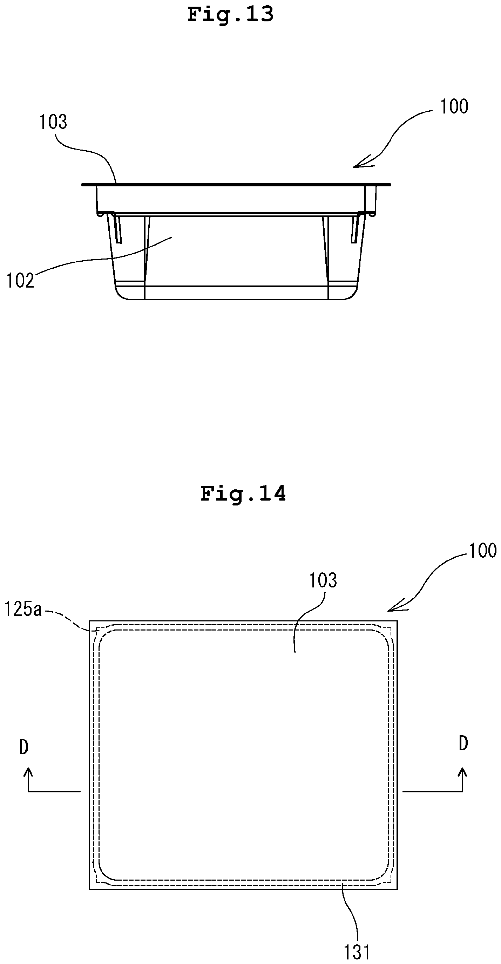

Embodiments of a package, shown in the drawings, which accommodates a plurality of the assemblies of the present invention for syringes are described below by using Figs. 11 through 15.

A package 100 of the present invention for the assembly for the prefilled syringe, accommodating a plurality of assemblies for syringes, which can be or is subjected to sterilization has a container body 102 whose upper surface is open and which has shape retainability, a barrel holding member 104 capable of holding a plurality of assemblies 10 for syringes accommodated inside the container body 102, a plurality of the assemblies 10 for syringes held by the barrel holding member 104, and a sheet-shaped lid member 103 which airtightly seals the open upper surface of the container body 102 and is peelable from the container body.

The package 100 of the present invention for the assembly for the prefilled syringe can be or is subjected to sterilization. As a sterilization method, the high-pressure steam sterilization, radiation or electron beam sterilization, and the ethylene oxide gas sterilization are used.

As shown in Figs. 11, 12, and 15, the package 100 of the present invention for the assembly for the prefilled syringe has the container body 102, the barrel holding member 104 capable of holding a plurality of assemblies 10 for syringes, a plurality of the assemblies 10 for syringes held by the barrel holding member 104, and the sheet-shaped lid member 103 which airtightly seals the open upper surface of the container body 102 and is peelable therefrom. The package 100 further includes an air-permeable part, having bacteria impermeability and sterilizing gas permeability, which is provided on the container body 102 or on the sheet-shaped lid member 103.

As shown in Figs. 11 through 15, the container body 102 is tray-shaped and has a strength and shape retainability to some extent and a predetermined depth. The container body 102 has a body part 121, a barrel holding member-holding portion 126, formed at an upper portion of the body part 121, for holding a peripheral portion of the barrel holding member 104 which holds a plurality of the assemblies 10 for syringes, and an annular flange 124 formed at the opening of the upper surface of the container body.

An annular heat-sealing convex portion 125 is formed on the upper surface of the annular flange 124 to fix the sheet-shaped lid member 103 to the heat-sealing convex portion 125. The barrel holding member-holding portion 126 is formed at a position located below the flange 124 by spacing it at a predetermined interval from the flange 124. In the container body 102 of a first embodiment, the barrel holding member-holding portion 126 is formed as an annular stepped portion so that the peripheral portion of the barrel holding member 104 which holds a plurality of the assemblies 10 for syringes can be placed thereon.

It is preferable that the container body 102 has shape retainability and rigidity to some extent. To respond to the high-pressure steam sterilization, it is desirable to use a thermoplastic material having heat resistance (not less than 120 degrees C). As materials having the shape retainability and the rigidity to some extent, heat resistance, and thermoplasticity, it is possible to exemplify polyolefin such as polypropylene and polyethylene, vinyl chloride resin, polystyrene/polypropylene resin, polyethylene/ionomer (for example, ethylene-based, styrene-based, fluorine-based)/polyethylene, polyester resin (for example, polyethylene terephthalate, polybutylene terephthalate, and amorphous polyethylene terephthalate), and PP/EVOH/PP (laminate) are listed. In this case, the thickness of the container 102 is set to favorably 0.05 to 4.00mm and especially favorably 1.00 to 2.00mm.

It is possible to subject the container body 102 to radiation sterilization or electron beam sterilization. In this case, it is desirable to use radiation-resistant material. As the radiation-resistant material (for example, radiation-resistant polyolefin), it is possible to use polyolefin (for example, polypropylene, polyethylene) to which hindered amine, an antioxidant, a nucleating agent, and the like are added to impart radiation resistance thereto. As the hindered amine, bis (2,2,6,6-tetramethylpiperidyl) sebacate, bis (2,2,6,6-tetramethylpiperidyl) adipate, bis(2,2,6,6-tetramethylpiperidyl)fumarate are exemplified. As the antioxidant, 1,1,3-tris(2-methyl-hydroxy-5-t-butylphenyl)butane, tris(3,5-di-T-butyl-4-hydroxybenzyl) isocyanurate, tetrakis(methylene-3-(3,5-di-t-butyl-4-hydroxyphenyl) propionate)methane are exemplified. As the nucleating agent, 1,3,2,4-dibenzylidene sorbitol and 1,3,2,4-di(p-methylbenzylidene) sorbitol are exemplified.

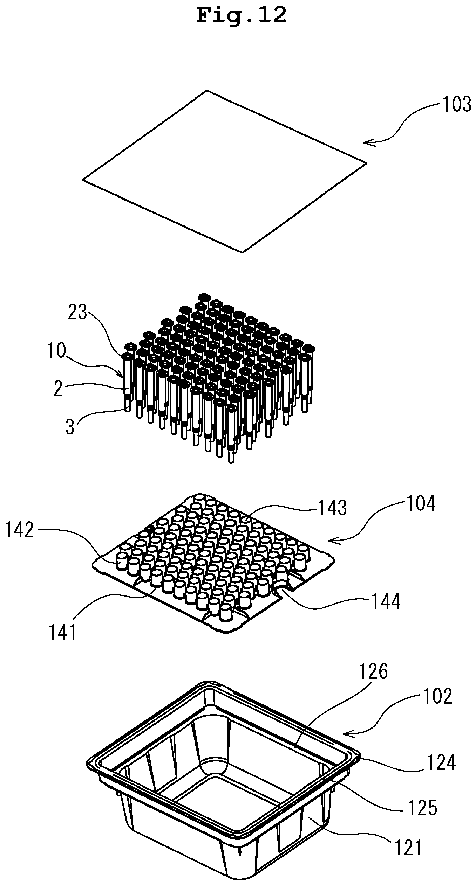

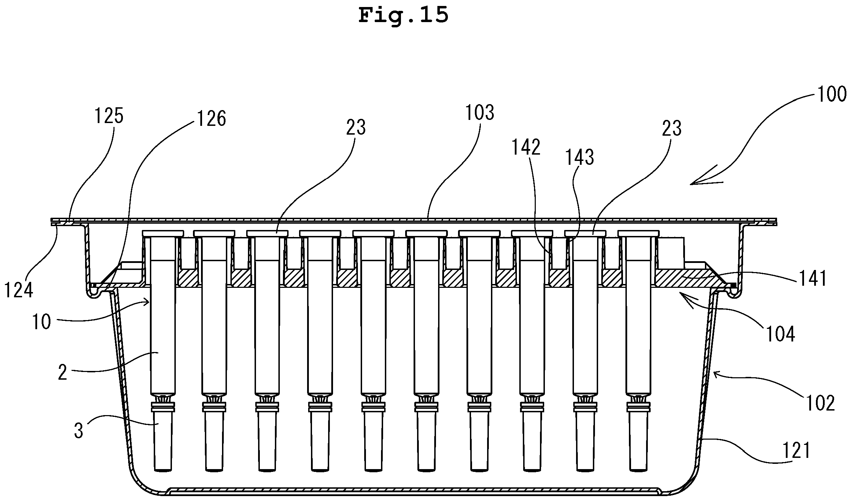

As shown in Figs. 12 and 15, the barrel holding member 104 capable of holding a plurality of the assemblies 10 for syringes has a substrate part 141 and a plurality of cylindrical parts 142 projecting upward from the substrate part 141. A barrel holding open portion 143 is formed inside each tubular part 142. A gripping cut-out 144 is formed on sides of the substrate part 141. The inner diameter of the tubular part 142 and that of the barrel holding open portion 143 are set larger than the outer diameter of a maximum diameter portion of the assembly 10 for the syringe held by the barrel holding member. It is impossible for the flange 23 of the assembly 10 for the syringe held by the barrel holding member to pass through the tubular part 142 and the barrel holding open portion 143.

Therefore, as shown in Fig. 15, the assembly 10 for the syringe passes through the cylindrical part 142, and the flange 23 of the assembly 10 for the syringe is hung by the barrel holding open portion 143. As shown in Fig. 15, the lower end (the distal end of the seal cap 3) of the assembly 10 for the syringe held by the barrel holding member 104 does not contact the bottom surface of the container body 102. In other words, the bottom surface of the container body 102 and the lower end (distal end of the seal cap 3) of the assembly 10 for the syringe held by the barrel holding member 104 are spaced away from each other so that the space therebetween does not inhibit the circulation of water vapor. It is desirable that a material for forming the barrel holding member 104 has heat resistance (not less than 120 degrees C) so that the barrel holding member is capable of responding to the high-pressure steam sterilization.

It is desirable that the sheet-shaped lid member 103 has a property of not allowing fine particles such as bacteria and virus to permeate therethrough to perform the high-pressure steam sterilization or the ethylene oxide gas sterilization and has a sterilizing gas-permeable property of allowing a sterilizing gas such as water vapor and ethylene oxide gas to permeate therethrough. It is preferable that the sheet-shaped lid member 103 can be heat-sealed to the container body 102. For example, it is possible to suitably use nonwoven cloth made of synthetic resin for the sheet-shaped lid member. More specifically, it is possible to use nonwoven cloth, known as Tyvek (registered trademark), which is made of a synthetic resin material such as polyolefin and a porous film made of synthetic resin for the sheet-shaped lid member.

The peripheral portion of the sheet-shaped lid member 103 is peelably heat-sealed to the heat-sealing convex portion 125 formed on the annular flange 124 of the container body 102. In the first embodiment, the outer edge of the sheet-shaped lid member 103 is not heat-sealed to the annular flange 124 of the container body 102 to easily peel the sheet-shaped lid member from the container body. A projected portion 125a formed at each corner of the heat-sealing convex portion 125 functions as a peeling starting portion. The thickness of the sheet-shaped lid member 103 is set to favorably 0.05 to 1.00mm and more favorably 0.10 to 0.50mm.

In the first embodiment, the air-permeable part is provided on the sheet-shaped lid member 103. The air-permeable part-forming position is not limited to the sheet-shaped lid member, but may be formed on the container body 102.

In the assembly for the syringe of the present invention, the seal cap (includes the pierceable part) is water vapor permeable. At least the pierceable part of the seal cap is formed of the elastic material containing silicone rubber as its main material, silicone particles, and a lubricant. Thus, when the seal cap is removed from the piercing needle pierced into the seal cap after a certain period of time lapses, the material forming the pierceable part of the seal cap does not stick to the outer surface of the piercing needle.

## Claims

1. A seal cap (3) for a piercing needle-attached barrel (2) comprising a piercing needle-attached barrel (2) including a barrel body (21) having a piercing needle mounting portion (22) and a hollow piercing needle (6) which has a piercing needle tip (61) at a distal end thereof and whose proximal end portion is fixed to said piercing needle mounting portion (22);
wherein said seal cap (3) has a pierceable part (33) into which said piercing needle (6) can be pierced and is water vapor permeable,
**characterized in that**
at least said pierceable part (33) of said seal cap (3) is formed of an elastic material containing silicone rubber as a main material thereof, silicone particles, and a lubricant.

2. The seal cap (3) for a piercing needle-attached barrel (2) according to claim 1, which is entirely formed of said elastic material.

3. The seal cap (3) for a piercing needle-attached barrel (2) according to claim 1 or 2, wherein said silicone particles are spherical silicone particles.

4. The seal cap (3) for a piercing needle-attached barrel (2) according to any one of claims 1 through 3, wherein said silicone particles are at least one kind selected from among silicone rubber particles, silicone resin particles, and silicone composite particles.

5. The seal cap (3) for a piercing needle-attached barrel (2) according to claim 1 through 4, wherein said silicone particles are at least one kind selected from among silicone rubber particles, silicone resin particles, and silicone composite particles consisting of silicone rubber powder whose surface is coated with silicone resin.

6. The seal cap (3) for a piercing needle-attached barrel (2) according to any one of claims 1 through 5, wherein said lubricant is at least one kind selected from among metal soap, higher fatty acid, higher aliphatic alcohol, higher aliphatic amine, and higher aliphatic amide.

7. The seal cap (3) for a piercing needle-attached barrel (2) according to claim 6, wherein said metal soap is at least one kind selected from among zinc stearate, calcium stearate, barium stearate, lithium stearate, magnesium stearate, calcium laurate, barium laurate, zinc laurate, calcium ricinoleate, barium ricinoleate, zinc ricinoleate, and zinc octylate.

8. The seal cap (3) for a piercing needle-attached barrel (2) according to any one of claims 1 through 7, wherein said silicone rubber contained in said elastic material is crosslinked.

9. The seal cap (3) for a piercing needle-attached barrel (2) according to any one of claims 1 through 8, said pierceable part (33) of said seal cap (3) is a silicone particle- added portion and a content of said silicone particle at said silicone particle- added portion is 0.5 to 10 wt%.

10. The seal cap (3) for a piercing needle-attached barrel (2) according to any one of claims 1 through 9, said pierceable part (33) of said seal cap (3) is a lubricant-added portion and a content of said lubricant at said lubricant-added portion is 0.3 to 5.0 wt%.

11. An assembly for a syringe comprising a piercing needle-attached barrel (2) including a barrel body (21) having a piercing needle mounting portion (22) and a hollow piercing needle (6) which has a piercing needle tip (61) at a distal end thereof and whose proximal end portion is fixed to said piercing needle mounting portion (22) and a seal cap (3) according to any of claims 1 through 5 for sealing said piercing needle tip (61);
wherein said piercing needle tip (61) has been pierced into said pierceable part (33) of said seal cap (3).

12. The assembly for a syringe according to claim 11, which is subjected to high-pressure steam sterilization.

13. The assembly for a syringe according to claim 11 or 12, wherein said seal cap (3) has a closed distal end part, an open proximal end part, a hollow part into which said piercing needle (6) can be inserted, and a pierceable part (33) into which said piercing needle tip (61) inserted into said hollow part can be pierced; and said open proximal end part of said seal cap (3) is mounted on a distal end portion of said barrel in an airtight state.

14. A prefilled syringe comprising an assembly for a syringe according to any one of claims 11 through 13, a gasket which is accommodated inside said barrel and liquid-tightly slidable inside said barrel body (21), and a medical agent filled inside a space formed of said piercing needle-attached barrel (2) and said gasket.

15. A package, for an assembly for a syringe, for accommodating a plurality of assemblies for syringes according to any one of claims 11 through 14, comprising;
a container body whose upper surface is open and which has shape retainability,
a barrel holding member capable of holding a plurality of said assemblies for syringes,
a plurality of said assemblies for syringes held by said barrel holding member, and
a sheet-shaped lid member which airtightly seals said open upper surface of said container body and is peelable therefrom, and
an air-permeable part, having bacteria impermeability and sterilizing gas permeability, which is provided on said container body or on said lid member, and
wherein said package is subjected to high-pressure steam sterilization.

## Patentansprüche

1. Dichtkappe (3) für eine mit einer Einstechnadel versehene Hülse (2), die eine mit einer Einstechnadel versehene Hülse (2) mit einem Hülsenkörper (21) mit einem Einstechnadel-Befestigungsabschnitt (22) und einer hohlen Einstechnadel (6) hat, die an ihrem distalen Endabschnitt eine Einstechnadelspitze (61) hat und deren proximaler Endabschnitt an dem Einstechnadel-Befestigungsabschnitt (22) befestigt ist;
wobei die Dichtkappe (3) einen durchstechbaren Teil (33) aufweist, in den die Einstechnadel (6) eingesteckt werden kann und der wasserdampfdurchlässig ist,
**dadurch gekennzeichnet, dass**
zumindest der durchstechbare Teil (33) der Dichtkappe (3) aus einem elastischen Material ausgebildet ist, das Silikonkautschuk als Hauptmaterial, Silikonpartikel und ein Schmiermittel enthält.

2. Dichtkappe (3) für eine mit einer Einstechnadel versehene Hülse (2) gemäß Anspruch 1, die vollständig aus dem elastischen Material ausgebildet ist.

3. Dichtkappe (3) für eine mit einer Einstechnadel versehene Hülse (2) gemäß Anspruch 1 oder 2, wobei die Silikonpartikel kugelförmige Silikonpartikel sind.

4. Dichtkappe (3) für eine mit einer Einstechnadel versehene Hülse (2) gemäß einem der Ansprüche 1 bis 3, wobei die Silikonpartikel mindestens eine Art sind, die aus Silikonkautschukpartikeln, Silikonharzpartikeln und Silikonverbundpartikeln ausgewählt ist.

5. Dichtkappe (3) für eine mit einer Einstechnadel versehene Hülse (2) gemäß Anspruch 1 bis 4, wobei die Silikonpartikel mindestens eine Art sind, die aus Silikonkautschukpartikeln, Silikonharzpartikeln und Silikonverbundpartikeln ausgewählt ist, die aus Silikonkautschukpulver bestehen, dessen Oberfläche mit Silikonharz beschichtet ist.

6. Dichtkappe (3) für eine mit einer Einstechnadel versehene Hülse (2) gemäß einem der Ansprüche 1 bis 5, wobei das Schmiermittel mindestens eine Art ist, die aus Metallseife, höherer Fettsäure, höherem aliphatischem Alkohol, höherem aliphatischem Amin und höherem aliphatischem Amid ausgewählt ist.

7. Dichtkappe (3) für eine mit einer Einstechnadel versehene Hülse (2) gemäß Anspruch 6, wobei die Metallseife mindestens eine Art ist, die ausgewählt ist aus Zinkstearat, Calciumstearat, Bariumstearat, Lithiumstearat, Magnesiumstearat, Calciumlaurat, Bariumlaurat, Zinklaurat, Calciumricinoleat, Bariumricinoleat, Zinkricinoleat und Zinkoctylat.

8. Dichtkappe (3) für eine mit einer Einstechnadel versehene Hülse (2) gemäß einem der Ansprüche 1 bis 7, wobei der in dem elastischen Material enthaltene Silikonkautschuk vernetzt ist.

9. Dichtkappe (3) für eine mit einer Einstechnadel versehene Hülse (2) gemäß einem der Ansprüche 1 bis 8, wobei der durchstechbare Teil (33) der Dichtkappe (3) ein mit Silikonpartikeln versehener Abschnitt ist und der Gehalt an Silikonpartikeln in dem mit Silikonpartikeln versehenen Abschnitt 0,5 bis 10 Gew.-% beträgt.

10. Dichtkappe (3) für eine mit einer Einstechnadel versehene Hülse (2) gemäß einem der Ansprüche 1 bis 9, wobei der durchstechbare Teil (33) der Dichtkappe (3) ein mit Schmiermittel versetzter Abschnitt ist und ein Gehalt des Schmiermittels in dem mit Schmiermittel versetzten Abschnitt 0,3 bis 5,0 Gew.-% beträgt.

11. Baugruppe für eine Spritze, die eine mit einer Einstechnadel versehene Hülse (2) mit einem Hülsenkörper (21) mit einem Einstechnadel-Befestigungsabschnitt (22) und einer hohlen Einstechnadel (6), die an ihrem distalen Ende eine Einstechnadelspitze (61) aufweist und deren proximaler Endabschnitt an dem Einstechnadel-Befestigungsabschnitt (22) befestigt ist, und eine Dichtkappe (3) gemäß einem der Ansprüche 1 bis 5 zum Abdichten der Einstechnadelspitze (61) hat;
wobei die Einstechnadelspitze (61) in den durchstechbaren Teil (33) der Dichtkappe (3) eingestochen wurde.

12. Baugruppe für eine Spritze gemäß Anspruch 11, die einer Hochdruck-Dampfsterilisation unterzogen wird.

13. Baugruppe für eine Spritze gemäß Anspruch 11 oder 12, wobei die Dichtkappe (3) einen geschlossenen distalen Endabschnitt, einen offenen proximalen Endabschnitt, einen hohlen Teil, in den die Einstechnadel (6) eingesetzt werden kann, und einen durchstechbaren Teil (33) aufweist, in den die in den hohlen Teil eingesetzte Einstechnadelspitze (61) eingestochen werden kann; und wobei der offene proximale Endabschnitt der Dichtkappe (3) an einem distalen Endabschnitt der Hülse in einem luftdichten Zustand angebracht ist.

14. Vorgefüllte Spritze mit einer Baugruppe für eine Spritze gemäß einem der Ansprüche 11 bis 13, einer Dichtung, die im Inneren der Hülse untergebracht ist und flüssigkeitsdicht im Inneren des Hülsenkörpers (21) verschiebbar ist, und einem medizinischen Wirkstoff, der in einen Raum gefüllt ist, der aus der mit der Einstechnadel verbundenen Hülse (2) und der Dichtung ausgebildet ist.

15. Verpackung für eine Baugruppe für eine Spritze zur Aufnahme einer Vielzahl von Baugruppen für Spritzen gemäß einem der Ansprüche 11 bis 14, mit;
einen Behälterkörper, dessen obere Fläche offen ist und der formstabil ist,
ein Hülsenhalteelement, das in der Lage ist, eine Vielzahl der genannten Baugruppen für Spritzen zu halten,
eine Vielzahl der Anordnungen für Spritzen, die von dem HülsenHalteelement gehalten werden, und
ein blattförmiges Deckelelement, das die offene Oberseite des Behälterkörpers luftdicht verschließt und von diesem abziehbar ist, und
ein luftdurchlässiges Teil mit Bakterienundurchlässigkeit und Sterilisationsgasdurchlässigkeit, das an dem Behälterkörper oder an dem Deckelelement bereitgestellt ist, und
wobei die Verpackung einer Hochdruck-Dampfsterilisation unterzogen wird.

## Revendications

1. Capuchon d'étanchéité (3) pour un corps de seringue avec aiguille de ponction (2) comprenant un corps de seringue avec aiguille de ponction (2) incluant un corps de seringue (21) ayant une partie de montage d'aiguille de ponction (22) et une aiguille de ponction creuse (6) qui a une pointe d'aiguille de ponction (61) à une extrémité distale de celle-ci et dont la partie d'extrémité proximale est fixée à ladite partie de montage d'aiguille de ponction (22) ;
ledit capuchon d'étanchéité (3) ayant une partie perçable (33) dans laquelle ladite aiguille de ponction (6) peut percer et étant perméable à la vapeur d'eau,
**caractérisé en ce que**
au moins ladite partie perçable (33) dudit capuchon d'étanchéité (3) est formée d'un matériau élastique contenant du caoutchouc de silicone comme matériau principal, des particules de silicone et un lubrifiant.

2. Capuchon d'étanchéité (3) pour un corps de seringue avec aiguille de ponction (2) selon la revendication 1, qui est entièrement formé de ladite matière élastique.

3. Capuchon d'étanchéité (3) pour un corps de seringue avec aiguille de ponction (2) selon la revendication 1 ou 2, lesdites particules de silicone étant des particules de silicone sphériques.

4. Capuchon d'étanchéité (3) pour un corps de seringue avec aiguille de ponction (2) selon l'une quelconque des revendications 1 à 3, lesdites particules de silicone étant d'au moins une sorte choisie parmi des particules de caoutchouc de silicone, des particules de résine de silicone et des particules composites de silicone.

5. Capuchon d'étanchéité (3) pour un corps de seringue avec aiguille de ponction (2) selon l'une quelconque des revendications 1 à 4, lesdites particules de silicone étant au moins d'un type choisi parmi des particules de caoutchouc de silicone, des particules de résine de silicone et des particules composites de silicone constituées de poudre de caoutchouc de silicone dont la surface est recouverte de résine de silicone.

6. Capuchon d'étanchéité (3) pour un corps de seringue avec aiguille de ponction (2) selon l'une quelconque des revendications 1 à 5, ledit lubrifiant étant au moins d'un type choisi parmi un savon métallique, un acide gras supérieur, un alcool aliphatique supérieur, une amine aliphatique supérieure et un amide aliphatique supérieur.

7. Capuchon d'étanchéité (3) pour un corps de seringue avec aiguille de ponction (2) selon la revendication 6, ledit savon métallique étant au moins d'une sorte choisie parmi le stéarate de zinc, le stéarate de calcium, le stéarate de baryum, le stéarate de lithium, le stéarate de magnésium, le laurate de calcium, le laurate de baryum, le laurate de zinc, le ricinoléate de calcium, le ricinoléate de baryum, le ricinoléate de zinc, et l'octylate de zinc.

8. Capuchon d'étanchéité (3) pour un corps de seringue avec aiguille de ponction (2) selon l'une quelconque des revendications 1 à 7, ledit caoutchouc de silicone contenu dans ledit matériau élastique étant réticulé.

9. Capuchon d'étanchéité (3) pour un corps de seringue avec aiguille de ponction (2) selon l'une quelconque des revendications 1 à 8, ladite partie perçable (33) dudit capuchon d'étanchéité (3) étant une partie à particules de silicone ajoutées et une teneur de ladite particule de silicone dans ladite partie à particules de silicone ajoutées étant de 0,5 à 10 % en poids.

10. Capuchon d'étanchéité (3) pour un corps de seringue avec aiguille de ponction (2) selon l'une quelconque des revendications 1 à 9, ladite partie perçable (33) dudit capuchon d'étanchéité (3) étant une partie ajoutée de lubrifiant et une teneur dudit lubrifiant au niveau de ladite partie ajoutée de lubrifiant étant de 0,3 à 5,0 % en poids.

11. Ensemble pour une seringue comprenant un corps de seringue avec aiguille de ponction (2) comprenant un corps de seringue (21) ayant une partie de montage d'aiguille de ponction (22) et une aiguille de ponction creuse (6) qui a une pointe d'aiguille de ponction (61) à une extrémité distale de celle-ci et dont la partie d'extrémité proximale est fixée à ladite partie de montage d'aiguille de ponction (22) et un capuchon d'étanchéité (3) selon l'une quelconque des revendications 1 à 5 pour sceller ladite pointe d'aiguille de ponction (61) ;
ladite pointe d'aiguille de ponction (61) ayant percée dans ladite partie perçable (33) dudit capuchon d'étanchéité (3).

12. Ensemble pour une seringue selon la revendication 11, soumis à une stérilisation à la vapeur à haute pression.

13. Ensemble pour une seringue selon la revendication 11 ou 12, ledit capuchon d'étanchéité (3) ayant une partie d'extrémité distale fermée, une partie d'extrémité proximale ouverte, une partie creuse dans laquelle ladite aiguille de ponction (6) peut être insérée, et une partie perçable (33) dans laquelle ladite pointe d'aiguille de ponction (61) insérée dans ladite partie creuse peut percer ; et ladite partie d'extrémité proximale ouverte dudit capuchon d'étanchéité (3) étant montée sur une partie d'extrémité distale dudit corps de seringue à l'état étanche à l'air.

14. Seringue préremplie comprenant un ensemble pour une seringue selon l'une quelconque des revendications 11 à 13, un joint qui est logé à l'intérieur dudit corps de seringue et est coulissant de manière étanche au liquide à l'intérieur dudit corps de seringue (21), et un agent médical rempli à l'intérieur d'un espace formé par ledit corps de seringue avec aiguille de ponction (2) et ledit joint.

15. Emballage, destiné à un ensemble pour une seringue, destiné à recevoir une pluralité d'ensembles de seringues selon l'une quelconque des revendications 11 à 14, comprenant :
un corps de récipient dont la surface supérieure est ouverte et qui a une capacité de retenu de la forme,
un élément de maintien de corps de seringue capable de recevoir une pluralité desdits ensembles pour seringues,
une pluralité desdits ensembles pour seringues maintenus par ledit élément de maintien de corps de seringue, et
un élément de couvercle en forme de feuille qui scelle de manière étanche à l'air ladite surface supérieure ouverte dudit corps de récipient et qui peut être détaché de celui-ci, et
une partie perméable à l'air, ayant une imperméabilité aux bactéries et une perméabilité aux gaz stérilisants, qui est fournie sur ledit corps de récipient ou sur ledit élément de couvercle, et
ledit emballage étant soumis à une stérilisation à la vapeur à haute pression.
